# EUROPEAN PATENT SPECIFICATION

(11) **EP 4 007 653 B1**
(45) Date of publication and mention of the grant of the patent: **10.09.2025**
(21) Application number: 20753851.3
(22) Date of filing: 30.07.2020
(51) Int. Cl.: B01J 8/04

(54) **REVERSE FLOW REACTOR WITH RECUPERATIVE REVERSE-FLOW FEED CYCLE**
RÜCKFLUSSREAKTOR MIT REKUPERATIVEM RÜCKFLUSSSPEISEZYKLUS
RÉACTEUR À FLUX INVERSÉ À CYCLE D'ALIMENTATION À FLUX INVERSÉ RÉGÉNÉRATIF

(30) Priority: 02.08.2019 US 201962882033 P; 12.11.2019 EP 19208646
(43) Date of publication of application: 08.06.2022
(73) Proprietor: ExxonMobil Technology and Engineering Company, Spring, TX 77389 (US)
(72) Inventor: WEISS, Brian, M., Bridgewater, NJ 08807 (US); CHUN, Changmin, Raritan, NJ 08869 (US); LIU, Sophie, Hampton, NJ 08827 (US); BARRAI, Federico, Houston, TX 77006 (US); KEUSENKOTHEN, Paul, F., Houston, TX 77006 (US); YOUNG, Zachary, D., Houston, TX 77002 (US)
(74) Representative: ExxonMobil Petroleum & Chemical BV
(86) International application number: PCT/US2020/044246
(87) International publication number: WO 2021/025948

(56) References cited:
- WO-A1-2018/044548
- WO-A1-2018/044558
- WO-A1-2019/204082
- US-A1- 2007 144 940
- US-A1- 2013 317 801
- US-A1- 2015 065 767
- US-A1- 2017 137 285
- US-A1- 2019 153 331

## Description

### FIELD

This disclosure relates to reverse flow processes and reverse flow reactors. This disclosure is useful, e.g., in converting alkanes to form olefins.

### BACKGROUND

A reverse flow reactor (RFR) is known from, for example, US 2019/0153331 A1, US 2019/0055178 A1, WO 2018/044548 A1, US 9047439 B2, and US 7976797 B2. A key advantage of an RFR is that the product stream can be heat exchanged with a regeneration stream of air and flue gas as a diluent to recuperate heat and supply the enthalpy for the chemical reaction. However, the extent to which the product may be cooled in this way is limited by the ratio of regeneration flow to product flow, which is primarily determined by the heat capacity of the species in the streams. For example, when the regeneration stream is mainly oxygen and nitrogen, it typically has only 1/3 the mass specific heat capacity of a hydrocarbon such as ethane, and thus the regeneration mass flow must be about 3 times larger than the product flow to achieve sufficient cooling. Such a large regeneration flow is generally undesirable because the vessel(s) conducting the regeneration flow must be larger and/or more numerous than the vessel(s) conducting the hydrocarbon flow to avoid large pressure drops. As a result, the large air flow increases compression costs, equipment sizes, number of reactors and associated valves and other equipment.

What is needed is a way to reduce the large regeneration fluid flow, compression requirements, equipment sizes, and number of reactors and valves for a reverse flow reactor system.

US patent application publication 2015/0065767 describes a reverse flow process for converting alkanes to unsaturated hydrocarbons via an exothermic reaction, involving a forward-flow hydrocarbon conversion step and a reverse-flow hydrocarbon conversion step. Further reverse flow processes are described in US patent application publication 2007/0144940.

### SUMMARY

This summary is provided to introduce a selection of concepts that are further described below in the detailed description. This summary is not intended to identify key or essential features of the claimed subject matter, nor is it intended to be used as an aid in limiting the scope of the claimed subject matter.

It has been found, in a surprising manner, that less flow of the heat regeneration stream can be achieved in a reverse flow reactor (RFR) incorporating a reverse-flow feed cycle of heat recuperation from the product stream, in addition to the forward-flow feed cycle and the reverse flow heating or regeneration cycle. The recuperative cycle of reverse-flow feed allows a balancing between the specific heat capacities, substantially reducing the requirements for the regeneration stream. As a result, reactor volume and compression requirements are smaller, and there are further reductions in energy use, flue gas production, coke and CO formation, and CO₂ emissions.

The present invention provides a reverse flow reaction process according to claim 1.

Described herein but not part of the invention is a reverse flow reactor system, comprising: a plurality of parallel, flow-through reactor members comprising respective reaction zones; and a flow distribution system comprising one or more valves to: initiate a forward feed flow cycle wherein a forward flow of the feed through the reactor is heated and reacted in a forward reaction set of the reactor members to produce a forward reaction product; initiate a reverse feed flow cycle wherein a reverse flow of the feed through the reactor is heated and reacted in a reverse reaction set of the reactor members to produce a reverse reaction product; and initiate a regeneration cycle wherein a reverse flow of regenerant through the reactor heats and regenerates a regeneration set of the reactor members.

### BRIEF DESCRIPTION OF THE DRAWINGS

FIG. 1 is a simplified schematic diagram of a reverse flow reactor (RFR) cycle according to the prior art.
FIG. 2 is a simplified schematic piping diagram for an RFR reactor member adapted for a reverse feed flow cycle according to embodiments of this disclosure.
FIG. 3 is a simplified diagram of a cycle for the reactor member of FIG. 2 through the RFR stages according to embodiments of this disclosure.
FIG. 4 is a simplified schematic process flow diagram of an RFR system at one particular cycle stage discussed in Examples 6 and 7.
FIG. 5 is a simplified schematic diagram of an alumina substrate and a manganese oxide wash coat in a fabrication method according to an embodiment of this disclosure.
FIG. 6 is a simplified schematic diagram of an alumina substrate, a manganese-aluminum composite layer, and a washcoat of a manganese active phase, according to embodiments of this disclosure.
FIG. 7 is a simplified schematic diagram of an alumina substrate, a manganese-aluminum composite layer, and a washcoat of a manganese active phase doped with sodium tungstate promoter, according to embodiments of this disclosure.
FIG. 8 is an X-ray diffraction pattern of aluminum doped manganese oxide with sodium tungstate promoter according to an example of this disclosure, which shows reference lines for pure manganese oxide.
FIG. 9 is a cross sectional SEM image of an oxygenically active material according to an example of this disclosure.
FIG. 10 is a graph of reactor temperature versus operating cycles for a reverse flow reactor system according to an example of this disclosure and a comparative.

### DETAILED DESCRIPTION OF THE PREFERRED EMBODIMENTS

Throughout the entire specification, including the claims, the following terms shall have the indicated meanings. The words and phrases used herein should be understood and interpreted to have a meaning consistent with the understanding of those words and phrases by those skilled in the relevant art. No special definition of a term or phrase, i.e., a definition that is different from the ordinary and customary meaning as understood by those skilled in the art, is intended to be implied by consistent usage of the term or phrase herein. To the extent that a term or phrase is intended to have a special meaning, i.e., a meaning other than the broadest meaning understood by skilled artisans, such a special or clarifying definition will be expressly set forth in the specification in a definitional manner that provides the special or clarifying definition for the term or phrase.

For example, the following discussion contains a non-exhaustive list of definitions of several specific terms used in this disclosure (other terms may be defined or clarified in a definitional manner elsewhere herein). These definitions are intended to clarify the meanings of the terms used herein. It is believed that the terms are used in a manner consistent with their ordinary meaning, but the definitions are nonetheless specified here for clarity.

A/an: The articles "a" and "an" as used herein mean one or more when applied to any feature in embodiments and implementations of this disclosure described in the specification and claims. The use of "a" and "an" does not limit the meaning to a single feature unless such a limit is specifically stated. The term "a" or "an" entity refers to one or more of that entity. As such, the terms "a" (or "an"), "one or more" and "at least one" can be used interchangeably herein.

About: As used herein, "about" refers to a degree of deviation based on experimental error typical for the particular property identified. The latitude provided the term "about" will depend on the specific context and particular property and can be readily discerned by those skilled in the art. The term "about" is not intended to either expand or limit the degree of equivalents which may otherwise be afforded a particular value. Further, unless otherwise stated, the term "about" shall expressly include "exactly," consistent with the discussion below regarding ranges and numerical data. All numerical values within the detailed description and the claims herein are modified by "about" or "approximately" the indicated value, and take into account experimental error and variations that would be expected by a person having ordinary skill in the art.

And/or: The term "and/or" placed between a first entity and a second entity means one of (1) the first entity, (2) the second entity, and (3) the first entity and the second entity. Multiple elements listed with "and/or" should be construed in the same fashion, i.e., "one or more" of the elements so conjoined. Other elements may optionally be present other than the elements specifically identified by the "and/or" clause, whether related or unrelated to those elements specifically identified. Thus, as a non-limiting example, a reference to "A and/or B", when used in conjunction with open-ended language such as "comprising" can refer, in one embodiment, to A only (optionally including elements other than B); in another embodiment, to B only (optionally including elements other than A); in yet another embodiment, to both A and B (optionally including other elements).

As used herein in the specification and in the claims, "or" should be understood to have the same meaning as "and/or" as defined above. For example, when separating items in a list, "or" or "and/or" shall be interpreted as being inclusive, i.e., the inclusion of at least one, but also including more than one, of a number or list of elements, and, optionally, additional unlisted items. Only terms clearly indicated to the contrary, such as "only one of" or "exactly one of," or, when used in the claims, "consisting of," will refer to the inclusion of exactly one element of a number or list of elements. In general, the term "or" as used herein shall only be interpreted as indicating exclusive alternatives (i.e. "one or the other but not both") when preceded by terms of exclusivity, such as "either," "one of," "only one of," or "exactly one of".

Comprising: In the claims, as well as in the specification, all transitional phrases such as "comprising," "including," "carrying," "having," "containing," "involving," "holding," "composed of," and the like are to be understood to be open-ended, i.e., to mean including but not limited to. Only the transitional phrases "consisting of" and "consisting essentially of" shall be closed or semi-closed transitional phrases, respectively, as set forth in the United States Patent Office Manual of Patent Examining Procedures, Section 2111.03. Any device or method or system described herein can be comprised of, can consist of, or can consist essentially of any one or more of the described elements.

Ranges: Concentrations, dimensions, amounts, and other numerical data may be presented herein in a range format. It is to be understood that such range format is used merely for convenience and brevity and should be interpreted flexibly to include not only the numerical values explicitly recited as the limits of the range, but also to include all the individual numerical values or sub-ranges encompassed within that range as if each numerical value and sub-range is explicitly recited. For example, a range of about 1 to about 200 should be interpreted to include not only the explicitly recited limits of 1 and about 200, but also to include individual sizes such as 2, 3, 4, etc. and sub-ranges such as 10 to 50, 20 to 100, etc. Similarly, it should be understood that when numerical ranges are provided, such ranges are to be construed as providing literal support for claim limitations that only recite the lower value of the range as well as claims limitation that only recite the upper value of the range. For example, a disclosed numerical range of 10 to 100 provides literal support for a claim reciting "greater than 10" (with no upper bounds) and a claim reciting "less than 100" (with no lower bounds). In the figures, like numerals denote like, or similar, structures and/or features; and each of the illustrated structures and/or features may not be discussed in detail herein with reference to the figures. Similarly, each structure and/or feature may not be explicitly labeled in the figures; and any structure and/or feature that is discussed herein with reference to the figures may be utilized with any other structure and/or feature without departing from the scope of the present disclosure.

The term "active" refers to substance having an element or compound that participates as a reactant in a chemical reaction and may optionally have catalytic characteristics.

The term "alkane" means substantially saturated compounds containing hydrogen and carbon only, e.g., those containing ≤1% (molar basis) of unsaturated carbon atoms. The term alkane encompasses C₂ to C₆ linear, iso, and cyclo alkanes.

The term "C*ₙ*" hydrocarbon wherein n is a positive integer, e.g., 1, 2, 3, 4, or 5, means hydrocarbon having *n* carbon atom(s) per molecule.

The term "C_{*n*+}" hydrocarbon wherein n is a positive integer, e.g., 1, 2, 3, 4, or 5, means hydrocarbon having at least *n* carbon atom(s) per molecule.

The term "C_{*n*-}" hydrocarbon wherein n is a positive integer, e.g., 1, 2, 3, 4, or 5, means hydrocarbon having no more than *n* number of carbon atom(s) per molecule.

The term "cycle time" means the time from a first interval to the next first interval, including (i) intervening second, third, and/or fourth intervals and (ii) any dead-time between any pair of intervals.

The term "flow-through reactor" refers to a reactor design in which one or more reagents enter a reactor, typically an elongated channel or stirred vessel, at an inlet, flow through the reactor, and then a product mixture (including any unreacted reagents) is continuously or semi-continuously collected at an outlet. Flow-through reactors include continuous reactors, as well as semi-continuous reactors in which one phase flows continuously through a vessel containing a batch of another phase, e.g., fixed-bed reactors where a fluid phase passes through a solid phase of catalyst, reactant, active material, etc.

With respect to flow-through reactors, the term "region" means a location within the reactor, e.g., a specific volume within the reactor and/or a specific volume between a flow-through reactor and a second reactor, such as a second flow-through reactor. With respect to flow-through reactors, the term "zone", refers to a specific function being carried out at a location within the flow-through reactor. For example, a "reaction zone" or "reactor zone" is a volume within the reactor for conducting at least one of oxidative coupling, oxydehydrogenation, dehydrocyclization, and so on. Similarly, a "quench zone" or "quenching zone" is a location within the reactor for transferring heat from products of the catalytic hydrocarbon conversion, such as C₂₊ olefin.

The term "hydrocarbon" means compounds containing hydrogen bound to carbon, and encompasses (i) saturated hydrocarbon, (ii) unsaturated hydrocarbon, and (iii) mixtures of hydrocarbons, including mixtures of hydrocarbons (saturated and/or unsaturated) having different values of *n.*

The term "oxidant" means any oxygen-bearing material which, under the conditions in the reaction zone, yields oxygen for transfer to the oxygen storage material, for storage with and subsequent release from the oxygen storage material to the oxidative coupling and/or oxydehydrogenation. While not wishing to be limited to theory, molecular oxygen atoms may be provided as a reactive gas in a gaseous zone and/or atomic oxygen may be provided from a catalyst surface as, for instance, reacted, sorbed forms.

The terms "oxidized state" and "reduced state" refer to relative states of oxidation and reduction with respect to a reference state. For example, in compositions of the formulae Mn⁺²_{A1}Mn⁺³_{B1}Oₓ and Mn⁺²_{A2}Mn⁺³_{B2}O_{y}, where x<y, A1>A2, and B1<B2, Mn⁺²_{A1}Mn⁺³_{B1}Oₓ is the reduced state compound and Mn⁺²_{A2}Mn⁺³_{B2}O_{y} is the oxidized state compound.

The term "oxydehydrogenation" means oxygen-assisted dehydrogenation of an alkane, particularly a C₂₊ alkane, to produce an equivalent alkene and water.

The term "reaction stage" or "reactor stage" means at least one flow-through reactor, optionally including means for conducting one or more feeds thereto and/or one or more products away therefrom.

The term "residence time" means the average time duration for non-reacting (non-converting by oxidative coupling) molecules (such as He, N₂, Ar) having a molecular weight in the range of 4 to 40 to traverse the reactor or a defined zone within the reactor, such as a reaction zone of a oxidative coupling reactor.

The term "spinel" refers to the cubic crystalline structure of the spinel class of minerals typified by the mineral spinel, MgAl₂O₄, or a material having such a structure. A spinel has the general formula AB₂X₄, where X is an anion such as chalcogen, e.g., oxygen or sulfur, arranged in a cubic close-packed lattice, and A and B are cations, which may be different or the same, occupying some or all of the octahedral and tetrahedral sites in the lattice, also including the so-called inverse spinels where the B cations may occupy some or all of the typical A cation sites and vice versa. Although the charges of A and B in the prototypical spinel structure are +2 and +3, respectively, i.e., A²⁺B³⁺₂X²⁻₄, other combinations incorporating divalent, trivalent, or tetravalent cations, including manganese, aluminum, magnesium, zinc, iron, chromium, titanium, silicon, and so on, are also possible.

The term "unsaturated" means a C*ₙ* hydrocarbon containing at least one carbon atom directly bound to another carbon atom by a double or triple bond.

According to one aspect of this disclosure, embodiments provide a reverse flow reaction process. The process comprises a forward feed flow cycle, a reverse feed flow cycle, and a reverse regeneration cycle. The forward feed cycle comprises heating and reacting a forward flow of a feed through a flow-through reactor member to produce a first reaction product. The reverse feed follow cycle comprises heating and reacting a reverse flow of the feed through the reactor member in a reverse flow direction opposite the forward feed cycle to produce a second reaction product. The regeneration cycle comprises heating and regenerating the reactor member with a flow of regenerant through the reactor member in the reverse flow direction.

In another aspect, this disclosure provides a reaction process comprising: providing a reactor comprising a plurality of parallel, flow-through reactor members; a forward feed flow cycle comprising heating and reacting a forward flow of a feed through the reactor in a forward flow set of the reactor members to produce a first reaction product; a reverse feed flow cycle comprising heating and reacting a reverse flow of the feed through the reactor in a reverse flow set of the reactor members to produce a second reaction product; and a regeneration cycle comprising heating and regenerating a regeneration set of the reactor members with a reverse flow of regenerant through the reactor. The process can further comprise sequentially alternating ones of the reactor members between the forward reaction set in the forward feed flow cycle, the reverse reaction set in the reverse feed flow cycle, and the regeneration set in the regeneration cycle.

In any embodiment of the process, the reactor members can comprise first and second opposed apertures adjacent respective first and second ends of a reactor and flow passages through the respective reactor members between the first and second opposed apertures. The forward feed flow cycle can comprise establishing the forward flow of the feed from a first feed conduit to the first apertures of the forward reaction set of the reactor members, passing the forward flow of the feed through the reactor members in a forward direction to form a first reaction product, and establishing a flow of the first reaction product from the second apertures of the forward reaction set of the reactor members to a first reaction product conduit. The reverse feed flow cycle can comprise establishing the reverse flow of feed from a second feed conduit to the second apertures of the reverse reaction set of the reactor members, passing the reverse flow of feed through the reactor members in a reverse direction to form a second reaction product, and establishing a flow of the second reaction product from the first apertures of the reverse reaction set of the reactor members to a second reaction product conduit. The regeneration cycle can comprise establishing the reverse flow of the regenerant from a regenerant conduit to the second apertures of the regeneration set of the reactor members, passing the regenerant through the reactor members in a reverse direction, to regenerate the reactor members, and establishing a flow of regenerant effluent from the first apertures of the regeneration set of the reactor members to an oxidant-lean conduit.

In any embodiment of the process, the sequential alternation can be repeated a plurality of times. If desired, a purge cycle can follow each of the respective forward feed flow, reverse feed flow, and regeneration cycles, preferably wherein the purge cycles comprise passing an inert gas, preferably steam, through, or applying vacuum to, a respective purge set of the reactor members.

In any embodiment, the process can further comprise recuperating heat, from an effluent of the regenerant and from a reverse feed flow reaction product, into the forward flow of the feed; and recuperating heat, from a forward feed flow reaction product, into the reverse flow of regenerant and the reverse flow of the feed. Preferably, a temperature of the first reaction product exiting the reactor members is no more than 200 °C, more preferably no more than 150°C, and even more preferably no more than 100 °C, higher than a temperature of the forward flow of feed entering the reactor members, and/or a temperature of the second reaction product exiting the reactor members is preferably no more than 200 °C, more preferably no more than 150°C, and even more preferably no more than 100 °C, higher than a temperature of the reverse flow of feed entering the reactor members. A temperature swing between cycles is preferably no more than 50°C, more preferably no less than about 25°C, and even more preferably the temperature swing is about 35°C. Preferably, there is a balanced heat flow wherein convection in the reactor members in the forward feed flow cycle matches total convection in the reactor members in the reverse feed flow cycle and the regeneration cycle.

Preferably, a residence time of the forward flow of feed in the forward flow set of the reactor members is different, more preferably shorter, and even more preferably equal to or less than about 30% shorter than a residence time of the reverse flow of feed in the reverse flow set of the reactor members.

In any embodiment, the regenerant can comprise an oxygen-containing gas and fuel, and the regeneration cycle heating can comprise combustion of the fuel.

In any embodiment of the process, the reactor members can comprise an active material. Often, the feed reaction comprises reduction of the active material, reducing an active phase of the active material from an oxidized state to a reduced state, and the regeneration cycle heating comprises oxidation of the active material, oxidizing the active phase from the reduced state to the oxidized state. The regenerant can be any oxygen-containing gas, such as oxygen, oxygen-enriched air, air diluted with flue gas, and so on. Preferably, the oxygen-containing gas is air. The active material preferably comprises a transition metal oxide such as manganese oxide, more preferably wherein the oxidized state of the transition metal oxide comprises a spinel structure.

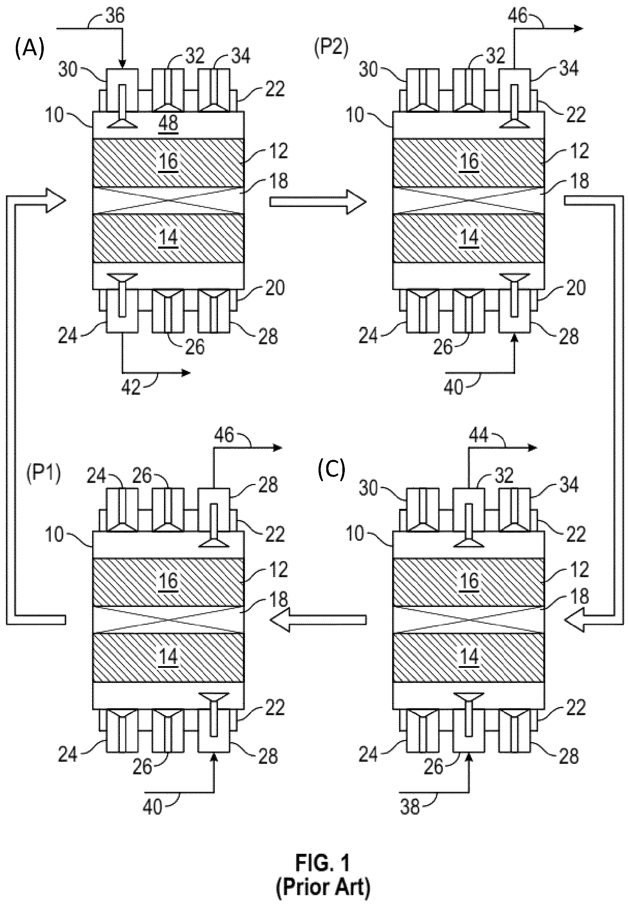

With reference to FIG. 1, in a conventional RFR, the reactor member 10 generally has a fixed bed 12 comprising heat exchange zones 14, 16, reaction zone 18, and first and second distributors 20, 22 comprising valves 24, 26, 28 and 30, 32, 34, respectively, which may comprise poppet valves or another suitable type of valve, used to regulate flows of all gases entering and exiting the reactor member 10. Another way to regulate the flows through the reactor member 10 is by rotating the bed or by using rotary valves, for example, as disclosed in WO200851606.

The reactor member 10 operates as a cyclic fixed bed reactor with alternating flows of feed such as HC 38 in forward feed cycle (A), oxidant such as oxygen in air 36 or another oxygen-containing gas in reverse regeneration cycle (C), and optional purge fluid flows such as steam 40 in purge cycles (P1), (P2). The other valves 24, 28, 30, 34 can remain closed.

Oxidation of a solid active material, and fuel combustion, are examples of the exothermic, regenerating reaction that can occur in reaction zone 18 in regeneration cycle (C). In any case, the air 36 (or other oxygen containing gas) is supplied via valve 30, preheated in heat exchange zone 16, exothermically reacted in reaction zone 18, and the resulting flue gas heats heat exchange zone 14 and is exhausted via valve 24 to flue gas line 42. During cycle (C), the other valves 26, 28, 32, 34 can remain closed.

Reforming, pyrolysis, hydropyrolysis, dehydrogenation, dehydrocyclization and hydroformylation are examples of the endothermic reactions that can occur in reaction zone 18 in forward feed cycle (A). The HC 38 or other feed as appropriate is supplied via valve 26, preheated in heat exchange zone 14, and endothermically reacted in reaction zone 18. The resulting product is quenched in heat exchange zone 16, and discharged via valve 32 to product line 44. If desired, the cycles (A) and (C) may be alternated with respective purge cycles (P1), (P2) in which an inert fluid such as steam 40 is introduced via valve 34, purges the bed 18, and is exhausted from valve 28 into return line 46. The purge cycles (P1), (P2) are optional, depending on the process, and can occur in the forward (in valve 28, out valve 34) and/or reverse (in valve 34, out valve 28) flow directions. A purge fluid other than steam can be any generally inert fluid such as nitrogen or argon, or a vacuum can be applied to one or both of valves 28, 34.

In cycle (A), HC 38 is flowed in the forward direction, and the high temperature in the reaction zone 18 induces an endothermic reaction such as cracking, and the reaction product is recovered via line 44. HC 38 is flowed until the bed 12 cools by a specified amount. Then, the bed 12 is purged with steam 40 in cycle (P1). Next, in cycle (C), a flow of air 36 (and fuel if applicable) generates heat and/or regenerates the reaction zone 18. When the temperature of the bed 12 swings by a specified amount, the flow of air 36 (and fuel if applicable) is stopped, and the bed 12 is purged with steam 40 in cycle (P2). Then the process repeats and starts anew with cycle (A).

An advantage of the RFR is that it recuperates heat in heat exchange zones 14, 16 and/or reaction zone 18, and uses that heat to supply the enthalpy for the endothermic chemical reaction. This allows more efficient use of energy than allowed by other reactors, such as a steam cracker, in which the heat in the product stream is used to boil water or oil. To accomplish heat recuperation, the prior art RFRs exchanged heat between a product stream and a regeneration stream that comprises air and flue gas recycle. Since the flue gas typically has just 1/3 the specific heat capacity of a hydrocarbon, the regeneration mass flow was about 3 times larger than the feed/product flow to achieve sufficient cooling. For example, for every reactor 10 in the forward feed conversion mode (A), three would be needed in reverse regeneration mode (C).

According to embodiments of this disclosure, both of the regeneration stream and a portion of the feed stream are supplied in a reverse direction to recuperate heat. In a cyclic RFR, this is accomplished by flowing a portion of the feed stream in a first or forward direction. After a specified period, this forward feed flow is stopped and the feed stream is flowed in a second or reverse direction that is opposite to the first direction. After a specified period, the reverse feed flow is stopped, and a regeneration flow is established also in the second or reverse direction. After the reverse regeneration flow, the cycle is started anew with the forward feed flow again. Purge steps may be optionally included between each step, e.g., by flowing steam, nitrogen, or another inert gas, or by establishing vacuum.

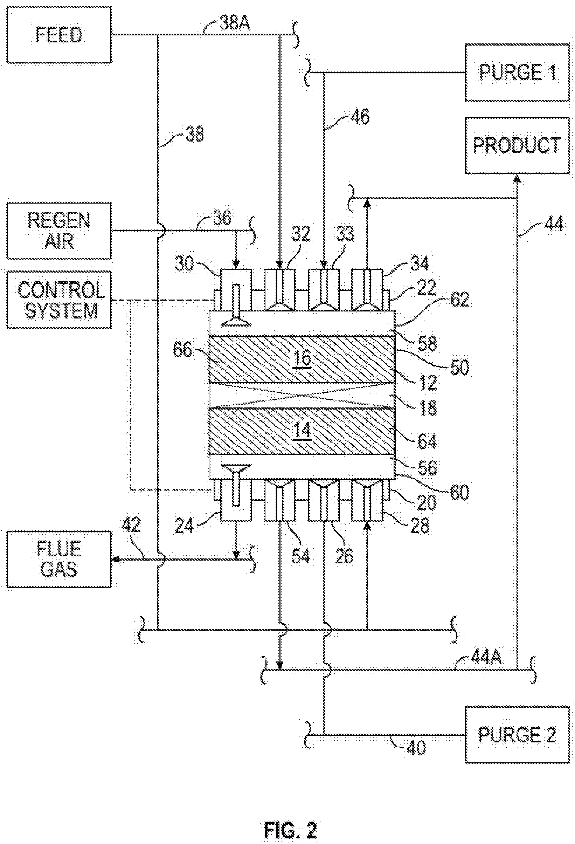

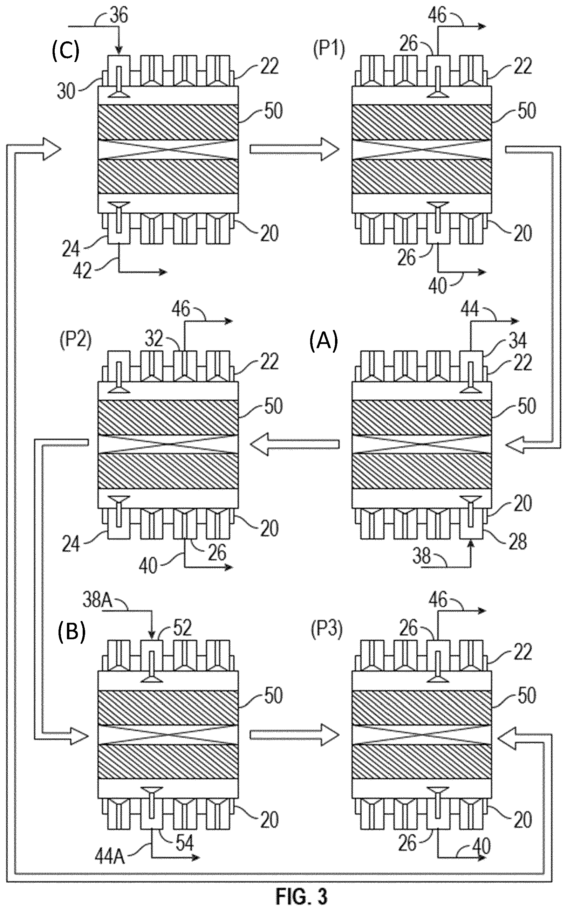

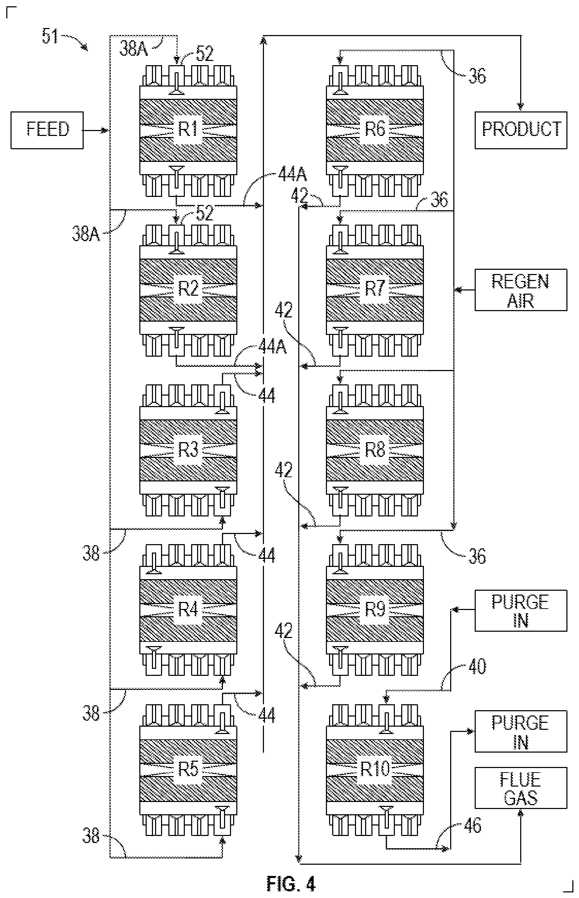

In embodiments of this disclosure, with reference to FIGs. 2-4, the inventive RFR system 51 (FIG. 4) comprises a plurality of parallel, flow-through reactor members R1 - R10. Each reactor member 50 (FIG. 2) comprises a reaction zone 18, and flow distributors 20, 22 comprising valves 24, 26, 28, 54 and 30, 32, 33, 34, respectively. The reverse regeneration cycle (C) (FIG. 3) can be initiated by opening valves 24 and 30 for a reverse flow of oxidant from line 36 through the reactor 50, and the forward feed cycle (A) can be initiated by opening valves 28, 34 for forward flow of feed from line 38, as discussed above in connection with FIG. 1. In addition, the reactor member 50 comprises valves 52 and 54 to initiate a reverse feed cycle (B) for a reverse-direction flow of feed from line 38A and product to line 44A.

As best seen in FIG. 2, the reactor member 50 has first and second opposed apertures 56, 58 adjacent respective first and second ends 60, 62. The bed 12 provides a flow passage 64 between the apertures 56, 58, and the heat exchange zones 14, 16 provide a heat absorbing material 66 fixed in the flow passage 64. The first distributor 20 for the first reactor end 60 comprises valves 28, 24, 54 to alternately provide fluid communication between the first aperture 56 and a forward feed conduit 38, or between the first aperture 56 and a flue gas conduit 42, or between the first aperture 56 and a reverse reaction product conduit 44A, respectively. The second distributor 22 for the second reactor end 62 comprises valves 34, 30, 52 to alternately provide fluid communication between the second aperture 58 and a forward reaction product conduit 44, or between the second aperture 58 and an oxidant conduit 36, or between the second aperture 58 and a reverse feed conduit 38A, respectively.

With reference to FIG. 3 and FIG. 2, in the forward feed cycle (A), the feed flows from the forward feed conduit 38, through the distributor 20, valve 28, and aperture 56, to pass through the heat exchange zone 14 where it is preheated, and then through the reaction zone 18 to form a forward reaction product. The forward reaction product then flows through the heat exchange zone 16 where it is cooled, then through second aperture 58 and valve 34 in distributor 22 and to forward reaction product conduit 44.

In the reverse regeneration cycle (C) in FIG. 3 and FIG. 2, which can sequentially follow the forward feed cycle (A) (and/or optional purge cycle (P1)), the air flows from the regeneration air conduit 36, through the distributor 22, valve 30, and aperture 58, to pass through the heat exchange zone 16 where it is preheated, and then through the reaction zone 18 to form a hot flue gas product. The hot flue gas flows through and heats the heat exchange zone 14, then through first aperture 56 and valve 24 in distributor 22 and to flue gas product conduit 42.

In the reverse feed cycle (B) in FIG. 3 and FIG. 2, the feed flows from the reverse feed conduit 38A, through the distributor 22, valve 52, and aperture 58, to pass through the heat exchange zone 16 where it is preheated, and then through the reaction zone 18 to form a reverse reaction product. The reverse reaction product then flows through the heat exchange zone 14 where it is cooled, then through first aperture 56 and valve 54 in distributor 20, and to reverse reaction product conduit 44A. The reverse feed and product can be separately supplied and/or processed in downstream purification equipment (not shown), with respect to the forward feed and product, or they can be from a common source and/or processed together, as shown.

If desired, the cycles (A), (B), and (C) may be sequentially alternated with respective purge cycles (P1), (P2), (P3). The purge cycles (P1), (P2), (P3) are optional, depending on the process, and can occur in the forward (in valve 28, out valve 34) and/or reverse (in valve 34, out valve 28) flow directions. A purge fluid other than steam can be any generally inert fluid such as nitrogen or argon, or a vacuum can be applied to one or both of valves 28, 34.

As stated above, FIG. 4 shows an RFR system 51 comprising ten reactor members R1 - R10. In this system, the two reactors R1, R2 are in reverse feed cycle mode, the three reactors R3, R4, R5 are in forward feed cycle mode, the four reactors R6, R7, R8, R9 are in regeneration mode.

In this disclosure, the ratios of the forward and reverse feed flows and the reverse regeneration flow depend on several factors. Generally, the feed stream is a hydrocarbon that undergoes an endothermic reaction that consumes heat. The oxygen in the reverse regeneration stream oxidizes a species to generate heat. A key factor that determines the flows in an RFR is the balance required between the heat supplied by oxidation and the demand of the endothermic reaction. Additional heat may also be required because the effluent streams may exit the reactor at higher temperature than the feed streams entering the reactor. It is desirable to limit the temperature rise between the effluent and feed streams to equal to or less than 200 °C, or even equal to or less than 150 °C or 100 °C. Furthermore, it is desirable to adjust the air flow to minimize excess oxygen in the process, so that the oxygen level in the effluent regeneration stream is preferably equal to or less than 5 vol %, or more preferably equal to or less than 2 vol % or 1 vol %. Often, based on relative specific heats, the desired flow of the regeneration stream is considerably less than the amount required to recuperate the heat from the product back to the reactor. According to this disclosure, the excess need for heat recuperation is supplied by directing a portion of the feed flow in the same direction as the regeneration flow, i.e., in a reverse direction. The portion is most suitably chosen to be in a ratio with the remainder of the hydrocarbon flow that can be approximately reduced to small integer coefficients. For example, the portion of hydrocarbon may be about 1/3 or 1/4 of the total flow, with the remaining 2/3 or 3/4 of the hydrocarbon flow in a direction counter to the regeneration flow.

If desired, where the regeneration is based on combustion, the fuel for combustion can be the mixture of methane and hydrogen byproduct from pyrolysis, which is also referred to herein as tail gas. Since hydrogen ignites easily, the combustion location can be controlled by a fuel plenum/distributor and mixer (not shown) to keep the air and fuel segregated until they reach the reaction zone 18. Also, pure methane can be used as the fuel. Since methane is more difficult to ignite than hydrogen, this approach can mix methane and air in the headspace 48, and a catalyst in the reaction zone 18 can ignite the mixture.

Active materials can also be used in the reaction zone 18 as a source of heat by enabling chemical looping. The active materials can be fabricated in a shape of a honeycomb monolith, spheres, rings, etc., and placed or packed in the reaction zone 18.

Chemical looping can selectively transfer oxygen to the endothermic reaction, enabling oxidative reactions such as oxidative dehydrogenation (ODH), or driving the reaction equilibrium forward in some cases, and greatly simplifies reactor operation and downstream product handling. In this two-step process in ODH, for example, the active materials donate oxygen from a lattice to convert ethane to ethylene and water in the reactor. After the active material is reduced, it is regenerated to complete the redox cycle. This process scheme eliminates drawbacks of conventional ODH because an expensive air separation unit is no longer required, and the overall process is safer since ethane does not get mixed with oxygen. It also reduces energy consumption and CO₂ emission in comparison with steam crackers due to selective oxidation of hydrogen that provides energy needed for ethane dehydrogenation. Moreover, higher single pass yields of ethylene and in-situ hydrogen oxidation reduce the load for the downstream separation and purification steps. With proper design of the active materials to avoid over-oxidation of ethane or ethylene, the use of lattice oxygen in the active materials can also lead to higher ethylene selectively.

The active material preferably comprises an active phase, a support phase, and a composite phase intermediate the active and support phases.

In any embodiment, the active phase can comprise an oxide of a first element selected from transition metal elements. The transition metal oxide is reversibly oxidizable and/or reducible between oxidized and reduced states. The transition metal oxide may be present in the oxidized state, the reduced state, or a combination thereof. The support phase generally comprises an oxide of a second element selected from IUPAC Group 2-14 elements. The composite phase comprises a mixed metal oxide of the first element and the second element.

The oxidation state of the transition metal oxide is generally determined by temperature and oxygen partial pressure of the process conditions, and the transition metal oxide is present in the oxidized state, the reduced state, or a combination thereof. Any suitable transition metal oxide that is stable at high temperatures, economical, and environmentally benign, may be used in the active phase. For the purposes of clarity and convenience, the following discussion addresses manganese as a preferred example of the active phase transition metal.

In the oxidized state, manganese oxide can be any of a variety of manganese oxides including manganese (II) oxide (MnO), manganese (II, III) oxide (Mn₃O₄), manganese (III) oxide (Mn₂O₃), manganese (IV) oxide (MnO₂, a.k.a. manganese dioxide), manganese (VI) oxide (MnO₃), and manganese (VII) oxide (Mn₂O₇). In the reduced state, the manganese can be metallic (Mn(0)), or any lesser oxidized manganese oxide than the oxidized state. By way of example, the oxidized state of the manganese can be Mn₂O₃, and the reduced state can be MnO. The oxidized state of the transition metal oxide preferably comprises a spinel structure.

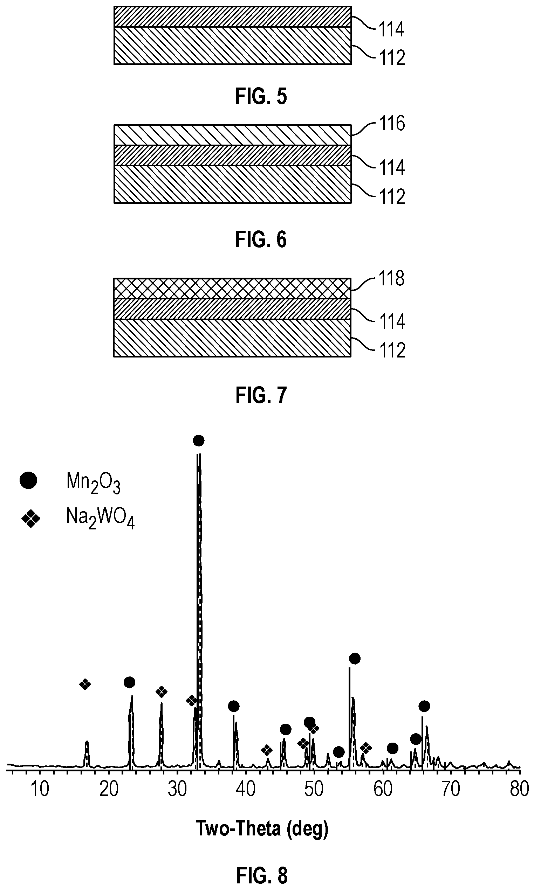

In any embodiment, the active phase can further comprise doping with the second element of the support phase. As a non-limiting example, aluminum-doped manganese oxide (1:10 Al:Mn) can be prepared by dissolving an appropriate quantity of aluminum isopropoxide into water, heating the mixture at 90 °C for 1 h while stirring, combining with an aqueous solution of manganese acetate, concentrating the resulting mixture at 90 °C, and then calcining the resulting gel at 900 °C for 2 h and cooling to ambient temperature. Optionally, sodium and tungsten can be added by dissolving a sodium source (e.g. sodium hydroxide), a tungsten source (e.g. tungstic acid), and optionally, an amine (e.g. triethanolamine) in water, adding the resulting solution to manganese oxide, and calcining at 900 °C for 2 h. The resulting Mn-Al-Na-W composite active material is characterized by having a diffraction pattern comprising a MnOx phase and optionally an Na₂WO₄ phase and an MnWO₄ phase. See FIG. 8.

The MnOₓ phase can be any of those listed above (e.g. Mn₂O₃, Mn₃O₄, MnO). The lattice constant of the MnOₓ phase is typically shifted from pure MnOₓ, which indicates incorporation of Al₂O₃ into the MnOₓ phase. For example, the MnOₓ phase may be Mn₂O₃ structure (space group #206) with a lattice constant of 9.33 A, which compares to a lattice constant of 9.41 A for pure Mn₂O₃. Optionally, the aluminum doped manganese oxide phase may be mixed with additional non-doped manganese material. In this case, the aluminum doped manganese oxide phase may comprise the material with the diffraction pattern described above. In another embodiment, the mixed aluminum-manganese phase may comprise the spinel structure, (Mn,Al)₃O₄. These phases may be co-mingled by standard methods, such as by ball-milling or mixing in a slurry phase. The manganese oxide active phase and/or composite phase can be applied on the support phase by a wash coating method. The manganese oxide active phase can be a fine oxide powder or precursors of the oxide phase. As non-limiting illustrative examples, the precursors can be carbonates, nitrates, sulfates, chlorides, alkoxides of metallic manganese. Fine oxide powder or precursors of the active oxide phase can be mixed to achieve a desired mass ratio of the active phase and the support phase.

One aspect of this disclosure is open porosity of the active material, which is preferably at the surface of the active phase. The volume percent of open pores in the active material is preferably in the range of 5 to 60 vol%, more preferably 10 to 50 vol%, and even more preferably 20 to 40 vol% of the active material. The open pores that are present in the active phase increase surface area of the active phase. The open pores are preferably connected and uniformly distributed in the active phase.

In the manganese oxide based active materials, the active phase is preferably present in the range of 1 to 20 wt% based on the total weight of the active material, including the active phase, support phase, and composite phase. Preferably, the active phase is in the range of 3 to 15 wt.%. More preferably, the active phase is in the range of 5 to 10 wt.%. (Optimum ranges may be adjusted later).

In any embodiment of this disclosure, the active phase can include a promoter, which can effectively suppress the undesired COₓ formation during the redox process in an oxidative dehydrogenation (ODH) reaction. A preferred ODH promoter is sodium tungstate (Na₂WO₄), which is mentioned in the following discussion for the purposes of illustrative simplicity and clarity. As non-limiting illustrative examples, the precursors of sodium tungstate can be carbonates, nitrates, sulfates, chlorides, alkoxides of metals comprising sodium and tungsten. The precursors of sodium tungstate can be mixed to achieve a desired mass ratio of the active phase. In the manganese oxide based active phase, the promoter is preferably in the range of 5 to 20 wt%, and more preferably in the range of 7 to 12 wt%, based on the total weight of the manganese oxide active phase.

In any embodiment of this disclosure, the active phase may further comprise a selectivity additive, e.g., cerium, to give preferential selectivity for hydrogen oxidation in an ODH system. As a non-limiting example, cerium nitrate in water is applied to Mn₃O₄ powder support for incipient wetness impregnation, and dried. Subsequently, a mixture of ammonium metatungstate and sodium tungstate in water can applied to the treated Mn₃O₄ powder for incipient wetness impregnation and again dried. The material (1:1 W:Ce by mole) can then calcined at 1100°C.

The support phase of the active material is a metal oxide (MₓO_{y}), wherein M is at least one metal selected from the group consisting of Al, Si, Mg, Ca, Sr, Ba, Sc, Y, La, Ce, Ti, Zr, Hf, and mixtures of Al, Si, Mg, Ca, Sr, Ba, Sc, Y, La, Ce, Ti, Zr, Hf, and/or Mn. The support phase is preferably a ceramic. As non-limiting illustrative examples, the support phase can be silica (SiO₂), magnesia (MgO), ceria (CeO₂), titania (TiO₂), zirconia (ZrO₂), cordierite (2MgO 2Al₂O₃ 2SiO₂), mullite (3Al₂O₃ 2SiO₂), aluminum titanate (Al₂TiO₅), magnesium aluminate (MgAl₂O₄), calcium-stabilized zirconia (CaO-ZrO₂), magnesium-stabilized zirconia (MgO-ZrO₂), yttria-stabilized zirconia (Y₂O₃-ZrO₂), yttria (Y₂O₃), barium zirconate (BaZrO₃), and strontium zirconate (SrZrO₃). Aluminum oxide (Al₂O₃, a.k.a. alumina) is a preferred support phase, and is referred to herein in the following discussion by way of example for the purposes of simplicity and clarity. Al₂O₃ can be in various crystallographic forms, including but not limited to, alpha-Al₂O₃, theta-Al₂O₃, and gamma-Al₂O₃.

The support phase can be any shape. Some non-limiting examples include honeycomb monoliths, foams, pellets, and beads in spherical, ellipsoidal, and polyhedral shapes. In a reverse flow reactor, a honeycomb monolith is a preferred shape of the support phase. In a fluidized bed reactor, a solid granular material is shaped as tiny spherical beads that can be suspended at high enough velocities and cause them to behave as though it were a fluid.

In any embodiment of this disclosure, the composite phase can comprise a mixed oxide including an oxide of the transition metal of the active phase and an oxide of the metal of the support phase, for example, a mixed oxide comprising both manganese oxide and alumina, such as (Mn,Al)₃O₄, which may help assure robustness of the active material. The intermediate composite layer can help accommodate any thermal expansion mismatch between the active phase and the support phase.

The composite phase preferably comprises a spinel structure, especially where the active phase comprises a spinel structure. For example, the composite phase can comprise (M¹, M²) spinel of the formula M¹_{A}M²_{B}O₄, wherein M¹ is the transition metal, M² is the second element, and A and B may range from 0.2 to 2.8 wherein the sum of A + B = 3. Preferably, M¹ is Mn and M² is Al, more preferably where A is about 1 and B is about 2.

Often, a liquid mixture comprised of both manganese and aluminum precursors or a slurry mixture comprising of both manganese oxide and alumina powder can be rapidly dried to form a dry mixed oxide powder by the use of a spray dryer. All spray dryers use some type of atomizer or spray nozzle to disperse the liquid mixture or the slurry mixture into a controlled drop size spray. Drop sizes from 10 µm to 500 µm can be achieved. The dry powder is often free flowing.

As one example, the composite phase of the mixed oxide dry powder can further be heat treated to form an MnAl₂O₄ and/or Mn₂AlO₄ spinel phase after sintering at the high temperatures which are often used to fabricate the support phase. Compared to a pure alumina support phase, an MnAl₂O₄ and/or Mn₂AlO₄ spinel phase serves as an intermediate phase between two completely differently manganese oxide and alumina phases. Use of such a mixed oxide support phase is particularly beneficial when the manganese oxide based active materials of this disclosure are prepared into a solid granular material to be adapted in a fluidized bed reactor. The mixed oxide particles provide a better thermal expansion match to a manganese oxide active phase and increase attrition resistance when they are flown through a fluidized bed reactor.

One aspect in this disclosure is to improve adhesion of the manganese oxide active phase on the support phase by a wash coating method. Due to the thermal expansion mismatch between manganese oxide and the alumina support phase, the wash coated manganese oxide tends to detach from the support phase. This issue can be overcome by forming an intermediate layer of the composite phase through a proper pretreatment method.

As a non-limiting example, with reference to FIGs. 5-7, a dense alumina support phase 112 is coated with an active phase Mn₃O₄ powder 114, with nominal thickness of about 5 µm. The construct is sintered at a temperature ranging from 1300°C to 1500°C. Due to this initial high temperature sintering, the Mn₃O₄ coating 114 is transformed into an (Mn,Al)₃O₄ composite phase and provides a better thermal expansion match to the alumina support phase. The intermediate (Mn,Al)₃O₄ composite phase 114 is not catalytically active, therefore, the thickness of this layer can be balanced between adhesion and functionality. After the high temperature sintering, adhesion can be assessed to determine whether the sintering temperature is sufficient to give the required reaction between the active phase and the support phase to form an intermediate (Mn,Al)₃O₄ composite phase 114. Then, a second wash coating 116 with an active phase Mn₃O₄ powder is applied on the intermediate (Mn,Al)₃O₄ composite layer 114 at thickness varying from 10~50 µm, and further sintered at a temperature ranging from 1000°C to 1300°C. Now the Na₂WO₄ promoter can be applied on the active Mn₃O₄ phase 116 to form the Mn-W active phase 118. Hence, one preferred embodiment of the manganese oxide based active materials 110 is comprised of manganese oxide active phase with an Na₂WO₄ promoter as a top layer 118, an intermediate (Mn,Al)₃O₄ composite phase 114, and alumina support phase as a bottom layer 112.

In any embodiment, the composite phase can comprise a plurality of graded phases comprising mixed oxides of both of the first and second elements having a successively relatively higher content of the first element adjacent the active phase and a successively relatively higher content of the second element adj acent the support phase. For example, where the support phase is an alumina substrate, and the active phase comprises a manganese oxide doped with alumina at a 10:1 molar ratio (MnAl_{0.1}Oₓ), the composite phase can comprise a first intermediate phase of MnAlOₓ (1:1 molar ratio Mn:Al) adjacent to the support phase, and a second intermediate phase of Mn₂AlOₓ (2:1 molar ratio Mn:Al) adjacent the active phase; or the composite phase may further comprise and a third intermediate phase of MnAl_{0.2}Oₓ (5:1 molar ratio Mn:Al) between the second intermediate phase and the active phase and a second intermediate phase adjacent the first intermediate phase of Mn₂AlOₓ (2:1 molar ratio Mn:Al); and so on.

### EXAMPLES

**Example 1 (Comparative):** A set of reverse flow reactors as shown in FIG. 1 is designed to react ethane by pyrolysis and tail gas combustion for regeneration. The reactor count is chosen as such to enable the same superficial gas velocity for ethane and air, which results in comparable hydraulic resistance for each stream. A cycle is set up such that ethane is flowed to 3 reactors and a regeneration gas comprising air and flue gas recycle is flowed to 9 reactors. The reactors require 6 valves each (2 for feed, 2 for regeneration, 2 for purge, see FIG. 1), and 72 valves total. Flow is cycled between each reactor, according to a sequential program such that each reactor experiences 3/12 of a cycle under ethane flow in an upward direction and 9/12 of a cycle under regeneration flow in a downward direction. To recuperate heat in this RFR, the total regeneration stream flow (1350 T/hr) is about 3.1 times the ethane flow (430 T/hr). The regeneration stream comprises a flow of 1350 T/h air, which results in a concentration of 11% O₂ in the flue gas. The results are summarized in Table 1.

**Example 2A: (Inventive):** A set of reverse flow reactors is designed to react ethane by pyrolysis in forward and reverse feed flow cycles and tail gas combustion for regeneration. A cycle is set up such that ethane is flowed to 3 reactors and a regeneration gas comprising air and flue gas recycle is flowed to 3 reactors. The reactor count is chosen as such to enable similar superficial gas velocity of the air and ethane streams, which results in comparable hydraulic resistance for each stream. Among the reactors flowing ethane, about 3/4 of the ethane or 320 T/h is flowed in an upward direction in 2 of these reactors and about 1/4 of the ethane or 110 T/h is flowed downward direction in one of these reactors. Flow is cycled between each reactor, according to a sequential program such that each reactor experienced 2/5 of a cycle under ethane flow in an upward direction, 1/5 of a cycle under ethane flow in a downward direction, and 2/5 of a cycle under regeneration flow in a downward direction. To recuperate heat in this RFR, the total regeneration stream flow (480 T/hr) is about 1.1 times the ethane flow (430 T/hr). The regeneration stream comprises a flow of 450 T/hr air, which results in a concentration of 2% O₂ in the flue gas. Notably, fewer reactors are required to conduct the regeneration flow at an equivalent hydraulic resistance versus Example 1. The results are summarized in Table 1.

**Example 2B: (Inventive):** A set of reverse flow reactors is designed to react ethane by pyrolysis in forward and reverse feed flow cycles and methane combustion for regeneration. A cycle is set up such that ethane is flowed to 3 reactors and a regeneration gas comprising air and flue gas recycle is flowed to 3 reactors. The reactor count is chosen as such to enable similar superficial gas velocity of the air and ethane streams, which results in comparable hydraulic resistance for each stream. Among the reactors flowing ethane, about 3/4 of the ethane or 320 T/hr is flowed in an upward direction in 2 of these reactors and about 1/4 of the ethane or 110 T/hr is flowed downward direction in one of these reactors. Flow is cycled between each reactor, according to a sequential program such that each reactor experienced 2/5 of a cycle under ethane flow in an upward direction, 1/5 of a cycle under ethane flow in a downward direction, and 2/5 of a cycle under regeneration flow in a downward direction. To recuperate heat in this RFR, the total regeneration stream flow (530 T/h) is about 1.1 times the ethane flow (430 T/h). The regeneration stream comprises a flow of 480 T/h air, which results in a concentration of 2% O₂ in the flue gas. Notably, fewer reactors are required to conduct the regeneration flow at an equivalent hydraulic resistance versus Example 1. The results are summarized in Table 1.

**Example 3 (Inventive):** A set of reverse flow reactors is designed to react ethane by oxidative dehydrogenation on an active material based on manganese oxide in forward and reverse feed flow cycles, and using chemical looping for reverse regeneration with air. The results are presented in Table 1 alongside similar results for examples 1 and 2 on the same basis.

**Table 1**

| **Example** | | **1** | **2A** | **2B** | **3** |
|---|---|---|---|---|---|
| **Flow scheme** | | Forward Feed/ Reverse Regeneration | Forward Feed/ Reverse Feed/ Regeneration | Forward Feed/ Reverse Feed/ Regeneration | Forward Feed/ Reverse Feed/ Regeneration |
| **Heat source** | | Tail gas combustion | Tail gas combustion | Methane combustion | Chemical looping |

| **Flows, T/h** | | | | | |
|---|---|---|---|---|---|
| | Ethane (forward) | 430 | 320 | 320 | 280 |
| | Ethane (reverse) | - | 110 | 110 | 150 |
| | Air flow (reverse)* | 1350 | 480 | 530 | 440 |
| **O₂ in flue gas, vol%** | | 11.0 | 2.0 | 2.0 | 2.0 |
| **Reactors on ethane** | | 3 | 3 | 3 | 3 |
| **Reactors on air** | | 9 | 3 | 3 | 3 |
| **Valves/reactor** | | 6 | 8 | 8 | 8 |
| **Valves, total** | | 72 | 48 | 48 | 48 |

| **Ratio** | | | | | |
|---|---|---|---|---|---|
| | Air / ethane (w/w) | | | | |
| | Convection (forward/reverse) | 3.1 | 1.1 | 1.2 | 1.0 |
| | Gas feed velocity (m/s / m/s) | 1.0 | 1.0 | 1.0 | 1.0 |

Although the original RFR of Example 1 exhibits many efficiencies, one inefficiency is the large air flow, which increases compression costs and equipment sizes. The improved, reverse-feed flow scheme shown for the RFR in Example 2 has a substantially reduced air flow since part of the HC is flowed in the reverse direction in an extra step of the cycle, and the overall weight ratio of air to ethane is reduced from 3.1 to just 1.2. As a result, part of the feed is warmed by heat recuperated from the quench. This configuration allows the air mass flow to stay at a level needed to stoichiometrically combust the fuel gas in Example 2, which, for ethane cracking, is close to the ethane mass flow. The substantial reduction of air/flue gas flow has a significant effect on equipment size and compressor cost, since these can now be much smaller. The chemical looping heat source in Example 3 uses approximately 9% less air (air:ethane w/w ratio = 1.1) because the chemical looping oxidative dehydrogenation combusts hydrogen (produced by dehydrogenating the ethane) instead of fuel gas, and H₂ has greater heat of combustion per O₂ consumed.

**Example 4 (Inventive):** A set of reverse flow reactors is designed to react ethane by pyrolysis in forward and reverse feed flow cycles and fuel gas combustion for regeneration. A cycle is set up such that ethane is flowed nominally up (forward cycle) to 3 reactors and down (reverse cycle) to 1 reactor, while a regeneration gas comprising air is flowed to 5 reactors, and 1 reactor is on purge with steam. The simulation assumed to have "ideal" temperature and reaction profiles where no reaction occurs in the heat exchanger zones, the reaction zone is isothermal, and the temperature swing is spread evenly throughout the reactor.

The pyrolysis flows were assigned fractional reactor counts to achieve 30% difference in residence time, where the fraction of time devoted to pyrolysis flow in the upward direction was adjusted to give ~30% less residence time versus flow in the downward direction. Flow continuity is maintained because common feed and product headers are used to respectively draw and deliver HC, and the change in flow rate could be achieved, for example, by modulating the lift height of poppet valves on the reactor. This feature allows pyrolysis to operate in a narrower conversion range throughout the cycle and extends the amount of time between purges, and thus reduces purge steam requirements.

The reactor bed is designed with a height that is 0.88 times the diameter, using alumina monoliths with a 35% open frontal area and 31 cells/cm² (200 cells/in.²). This obtained reaction residence times of 60 ms for the forward fed ethane, 80 ms for the reverse fed ethane, and 60 ms for the regeneration air; and preheat and quench heat exchanger residence times of 110 ms for the forward fed ethane, 110 ms for the reverse fed ethane, and 70 ms for the regeneration air. The The approach temperature is 40°C, and the reaction zone delta T is 50°C. The pressure drop is 29.0 kPa (4.2 psi) for the ethane feed in both directions, and 73.8 kPa (10.7 psi) for the regeneration air. These are acceptable pressure drops.

Cycle times were set at 15.0 s for the regeneration cycle, 8.4 s for the forward feed pyrolysis cycle, 3.6 s for the reverse feed pyrolysis cycle, and 3.0 s for all purge cycles, for a total cycle time of 30 s. The purge volumes were set based on the target O₂ impurity in the product effluent and the purge steam at the end of the purge, and the target ethane impurity in the flue gas and the purge steam at the end of the purge cycles. Longer cycle times are desirable in that less steam consumption in the purge cycles obtains from the lower number of purges per unit time. These cycle times are more than an order of magnitude longer than marine diesel engines, which provides a benchmark of commercially feasible cycle times using poppet valve technology. The results are presented in Table 4.

**Example 5 (Inventive):** A set of reverse flow reactors is designed to react ethane by pyrolysis in forward and reverse feed flow cycles and fuel gas combustion for regeneration as in Example 4, except that a longer reactor bed (L/D=1.06) is used; 2 pyroloysis reactors are fed up, 1 is fed down, 4 are regenerated, and 1 is on purge, for 8 total; and the bed material has 100 CPSI. As seen in Table 4 arrangement increases the reaction residence time of the reverse ethane flow and regeneration flow, increases the approach and reactor delta temperatures, increases the cycle time, reduces ethane losses, and reduces steam consumption.

**Example 6 (Inventive):** A set of reverse flow reactors is designed to react ethane by oxidative dehydrogenation (ODH) on manganese oxide in forward and reverse feed flow cycles, and to react air with the reduced manganese oxide for regeneration. A cycle is set up such that ethane is flowed up (forward cycle) to 3 reactors and down (reverse cycle) to 2 reactors, while a regeneration gas comprising air is flowed to 4 reactors, and 1 reactor is on purge with steam, without using any fractional reactor counts. The simulation assumed to have "ideal" temperature and reaction profiles as in Example 4.

The reactor bed is designed with a height that is 1.42 times the diameter, using alumina monoliths with a 35% open frontal area and 15.5 cells/cm² (100 cells/in.²) in the heat exchange zones. In the reaction zones, 6 mm pellets are loaded with 10 wt% Mn₃O₄ and have 60% voidage. The simulations of the RFR heat balance implied that 60-65% of the hydrogen product must be combusted to heat balance the RFR. Therefore, at 70% ethane conversion, the amount of active manganese oxide (Mn₃O₄) in the reactor should be in a ratio with the ethane flowed per cycle of ~0.45 Mn₃O₄ / ethane, by mole. These targets are achieved with only modest loadings of 10 wt% Mn₃O₄ in the central bed. The air flow was set to obtain 2.0 vol% oxygen in the flue gas.

This obtained reaction residence times of 360 ms for the forward fed ethane, 450 ms for the reverse fed ethane, and 320 ms for the regeneration air; and respective preheat and quench heat exchanger residence times of 200 ms and 100 ms for the forward fed ethane, 250 ms and 130 ms for the reverse fed ethane, and 120 ms and 100 ms for the regeneration air. The The approach temperature is 40°C, and the reaction zone delta T is 20°C. The pressure drop is 30.3 kPa (4.4 psi) for the ethane feed up, 25.5 kPa (3.7 psi) for the ethane feed down, and 67.6 kPa (9.8 psi) for the regeneration air. These are acceptable pressure drops.

Cycle times were set at 18.0 s for the regeneration cycle, 13.5 s for the forward feed pyrolysis cycle, 9.0 s for the reverse feed pyrolysis cycle, and 4.5 s for all purge cycles, for a total cycle time of 45 s. The purge volumes were set as in Example 4. The steam consumption was comparable to Examples 4 and 5, but the ethane losses were substantially lower than either Example 4 or Example 5. The results are also presented in Table 4.

**Example 7 (Inventive):** A set of reverse flow reactors is designed to react ethane by ODH on manganese oxide in forward and reverse feed flow cycles to react air with the reduced manganese oxide for regeneration as in Example 6, except that a shorter reactor bed (L/D=0.71) is used; the inert bed material has 200 CPSI; the active material comprises 8 wt% Mn₃O₄ and 50% voidage; and the residence and cycle times are shorter as shown in Table 2. As seen in Table 2, this arrangement marginally increases the ethane losses and steam consumption relative to Example 6.

**Table 2**

| **Example** | | **1** | **4** | **5** | **6** | **7** |
|---|---|---|---|---|---|---|
| **Heat source** | | Fuel gas combustion | | | Chemical looping | |
| **Feed up/down** | | 100% | 75%/25% | | 66%/33% | |
| **Reactors (no.)** | | **10** | **10** | **8** | **10** | **10** |
| | Feed (up) | 2 | 2.8 (3) | 2.1 (2) | 3 | 3 |
| | Feed (down) | 0 | 1.2 (1) | 0.9 (1) | 2 | 2 |
| | Regen (down) | 7 | 5 | 4 | 4 | 4 |
| | Purge | 1 | 1 | 1 | 1 | 1 |
| **Bed Height (L/D)** | | 0.88 | 0.71 | 1.06 | 1.42 | 0.71 |
| Inert form/material | | Monolith / Alumina | | | | |
| | OFA | 35% | 35% | 35% | 35% | 35% |
| | CPSI | 80 | 200 | 100 | 100 | 200 |
| **Active material** | | N/A | | | Pellets | |
| | Mn₃O₄ loading | | | | 10% | 8% |
| | Voidage | | | | 60% | 50% |

| **Reaction zone residence time, ms** | | | | | | |
|---|---|---|---|---|---|---|
| Ethane (up) | | 50 | 60 | 60 | 360 | 150 |
| Ethane (down) | | N/A | 80 | 90 | 450 | 200 |
| Regen (up) | | 40 | 60 | 60 | 320 | 130 |

| **Heat exchanger (preheat) residence time, ms** | | | | | | |
|---|---|---|---|---|---|---|
| Ethane (up) | | 70 | 110 | 100 | 200 | 120 |
| Ethane (down) | | N/A | 110 | 150 | 250 | 160 |
| Regen (up) | | 50 | 70 | 80 | 120 | 80 |

| **Heat exchanger (quench) residence time, ms** | | | | | | |
|---|---|---|---|---|---|---|
| Ethane (up) | | 40 | 110 | 50 | 100 | 60 |
| Ethane (down) | | N/A | 110 | 80 | 130 | 90 |
| Regen (up) | | 40 | 70 | 60 | 100 | 70 |
| **Approach temp** | | 150°C | 40°C | 60°C | 40°C | 40°C |
| **Reaction zone dT** | | 110°C | 50°C | 90°C | 20°C | 20°C |

| **Pressure drop, psi** | | | | | | |
|---|---|---|---|---|---|---|
| | Ethane (up) | 4.0 | 4.2 | 4.6 | 4.4 | 4.7 |
| | Ethane (down) | - | 4.2 | 3.5 | 3.7 | 3.7 |
| | Regen (up)* | 9.2 | 10.7 | 10.3 | 9.8 | 10.7 |

| **Temp. swing** | | 25°C | 35°C | 35°C | 35°C | 35°C |
|---|---|---|---|---|---|---|
| **Cycle time (total)** | | 22 s | 30 s | 35 s | 45 s | 30 s |
| | Regen (down) | 15.4 | 15.0 | 17.5 | 18.0 | 12.0 |
| | Pyro (up) | 4.4 | 8.4 | 9.2 | 13.5 | 9.0 |
| | Pyro (down) | | 3.6 | 3.9 | 9.0 | 6.0 |
| | Purge (all) | 2.2 | 3.0 | 4.4 | 4.5 | 3.0 |
| **Mn₃O₄ /C₂ /cycle** | | N/A | | | 0.5 mol/mol | 0.5 mol/mol |
| **Ethane in flue gas** | | 6 ppm (80 T/y) | 8 ppm (40 T/y) | 5 ppm (30 T/y) | 6 ppm (20 T/y) | 8 ppm (30 T/y) |
| **Steam flow** | | 0.25 | 0.28 | 0.20 | 0.25 | 0.30 |
| T steam / T HC | | | | | | |

| | | | | | | |
|---|---|---|---|---|---|---|
| N/A = Not Applicable | | | | | | |

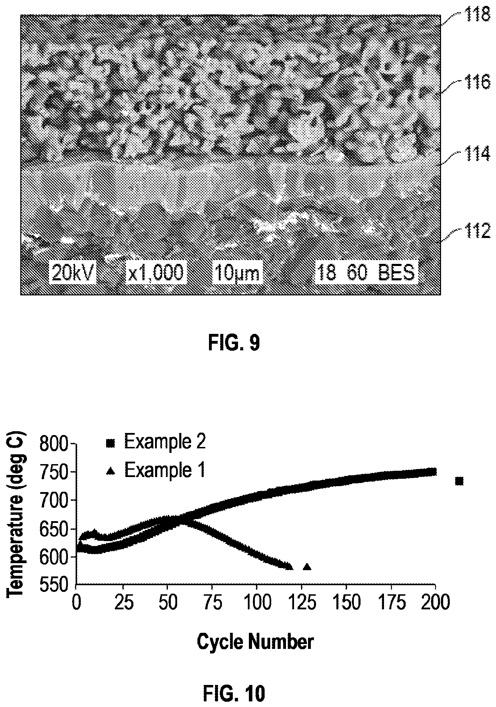

**Example 8:** In this example, the long-term retention of a washcoat on a composite phase was demonstrated. Manganese oxide based active materials were prepared by washcoating an Mn₃O₄ active phase on an alumina support phase with and without a composite phase. The washcoating was applied to alumina monoliths in the shape of honeycomb monoliths with 15.5 cells/cm² (100 cells/in.²), 50% open frontal area (OFA), 2.36 cm (6 in.) length, 6.35 cm (2.5 in.) diameter. In Example 1A (comparative), Mn₃O₄ (25 g) was washcoated onto a cylindrical alumina substrate. In Example 1B (inventive), 7 g of Mn₃O₄ were washcoated onto the cylindrical alumina substrate, the monolith was heated to 1450°C for 1 h, then subsequently, another 25 g Mn₃O₄ was washcoated onto the monolith, and the monolith was then heated to 1000°C for 1 h. FIG. 8 shows an X-ray diffraction (XRD) pattern of the aluminum doped manganese oxide with sodium tungstate promoter, and also shows the Mn₂O₃ reference lines for comparison. The Mn₂O₃ lattice contracts about 0.07 Å, indicating the Al incorporation. FIG. 9 shows a cross sectional scanning electron microscopy (SEM) of the corresponding aluminum doped manganese oxide with sodium tungstate promoter.

The washcoated monoliths were installed in a reverse flow reactor (RFR) between two alumina substrates (not washcoated) of 7.62 cm (3 in.) length but otherwise similar dimensions. The manganese oxide based active materials enabled redox reactions at fast time scales (~1-5 sec) with oxygen transfer to the lattice during regeneration step (high PO2, exothermic reaction) and oxygen transfer to the feed (low PO2, endothermic reaction) during the reaction step. The RFR was preheated to between about 600° and 650°C. At that point, a cycling program was established involving flows of 1) 50% hydrogen/nitrogen at 10 sLm in one direction for 6 seconds, 2) nitrogen at 5 sLm in the same direction for 6 seconds, 3) air at 25 sLm in the same direction for 6 seconds, and 4) nitrogen at 40 sLm in the opposite direction for 6 seconds. Feed gases were preheated to 300°C.

The temperature at the reactor center versus the number of complete cycles (steps 1 to 4) is shown in FIG. 9. For Example 8A, the temperature started increasing initially, indicating that heat was generated by combusting hydrogen with oxygen stored on the monolith, but then the temperature decreased after about 50 cycles indicating that heat was no longer generated by combusting hydrogen with oxygen stored on the monolith. Later analysis of the spent material showed that the washcoat had separated from the monolith and blocked flow through the channels. The washcoat detachment was associated with the poor performance of Example 1A. For Example 1B, the reactor temperature continued to increase due to hydrogen combustion from oxygen in the manganese. Compared to Example 8A, the loss of active phase was substantially reduced in Example 8B.

**Example 9:** This example demonstrates that alumina can be doped in the manganese active phase for long-term integrity without adversely impacting activity. In Example 9A, manganese(II) acetate tetrahydrate (25.92 g) was added to 720 mL water to form a manganese solution. Isopropanol (0.81 mL) was added to the manganese solution and heated at 85°C until a gel formed. The gel was calcined at 900°C for 20 h to form a manganese powder. In a separate container, 0.632 g sodium tungstate dihydrate and 0.67 g triethanolamine were added to 0.66 g water to form a tungsten solution. The tungsten solution was added to the manganese powder. The resulting material was heated at 900°C for 8 h.

For example 9B, aluminum isoproproxide (2.16 g) was added to 130 mL water and heated at 90°C for 1 h to form an aluminum suspension. In a separate container, 25.92 g manganese(II) acetate tetrahydrate was added to 720 mL water to form a manganese solution. The manganese solution was added to the aluminum suspension and the resulting mixture was heated at 90°C until a gel formed. The gel was calcined at 900°C for 20 h to form a manganese-aluminate powder. In a separate container, 0.632 g sodium tungstate dihydrate and 0.67 g triethanolamine were added to 0.66 g water to form a tungsten solution. The tungsten solution was added to the manganese aluminate powder. The resulting material was heated at 900°C for 8 h.

**TABLE 3**

| **Example:** | 9A | 9B |
|---|---|---|
| **Manganese oxide** | | |
| Al:Mn, mol/mol | 0:10 | 1:10 |

| **Product Species** | | |
|---|---|---|
| CO, dry mol% | 0.6 | 0.6 |
| CO2, dry mol% | 0.8 | 0.9 |
| H2, dry mol% | 18.7 | 18.8 |
| Methane, dry mol% | 9.0 | 9.6 |
| Ethane, dry mol% | 24.6 | 24.2 |
| Ethylene, dry mol% | 46.3 | 45.9 |

The materials of examples 9A and 9B were run in an ethane oxidation test. In each test, 0.4 grams of material was loaded in a quartz reactor and heated to 850 deg C while flowing 10% oxygen/nitrogen. The oxygen was purged with argon and a flow of 0.2 std L/min of ethane was established through the reactor. The concentrations of species in the products were measured and averaged over the first 8 seconds of ethane exposure. As seen in Table 3, the sample comprising alumina had similar performance to the base sample without alumina.

When the test is extended, the sample comprising alumina will have enhanced stability over long-term tests.

**Example 10:** In this example, alumina coupons were coated with inks of various manganese and aluminum oxides to investigate the integrity of the coating. The inks were prepared as follows:
*Ink A (MnAlOₓ):* Aluminum nitrate nonahydrate (303.86 g, 0.81 mol) and manganese(II) nitrate tetrahydrate (203.32 g, 0.81 mol) were dissolved in 500 mL water in a beaker under nitrogen sparge. In a separate beaker, ammonium carbonate (214.82 g, 2.24 mol) was dissolved in 1 L water. While stirring, the metal salt solution was added slowly to the ammonium carbonate solution. The mixture was allowed to stir for 2 h. The resulting precipitate was collected by vacuum filtration and washed with deionized water. The solid was transferred to an alumina crucible and dried in an oven at 110°C for 18 h and then calcined in a box furnace at 950°C for 15 h (5°C/min ramp) under air flow and then again after grinding for another 18 h followed by an additional calcination at 1300°C under static air for 24 h (5°C/min ramp and cool down).
*Ink B (Mn₂AlOₓ):* Aluminum nitrate nonahydrate (185.69 g, 0.495 mol) and manganese(II) nitrate tetrahydrate (248.50 g, 0.990 mol) were dissolved in 500 mL water in a beaker under nitrogen sparge. In a separate beaker, ammonium carbonate (184.10 g, 1.92 mol) was dissolved in 860 mL water. While stirring, the metal salt solution was added slowly to the ammonium carbonate solution. The mixture was allowed to stir for 2 h. The resulting precipitate was collected by vacuum filtration and washed with deionized water. The solid was transferred to a crucible and dried in an oven at 110 °C for 18 h and then calcined in a box furnace at 950 °C for 15 h (5 °C/min ramp) under air flow and then again after grinding for another 18 h followed by an additional calcination at 1300 °C under static air for 24 h (5 °C/min ramp and cool down).
*Ink C (MnAl_{0.2}Oₓ):* Aluminum nitrate nonahydrate (92.89 g, 0.25 mol) and manganese(II) nitrate tetrahydrate (310.62 g, 1.24 mol) were dissolved in 500 mL water in a beaker under nitrogen sparge. In a separate beaker, ammonium carbonate (170.0 g, 1.77 mol) was dissolved in 860 mL water. While stirring, the metal salt solution was added slowly to the ammonium carbonate solution. The mixture was allowed to stir for 2 h. The resulting precipitate was collected by vacuum filtration and washed with deionized water. The solid was transferred to a crucible and dried in an oven at 110 °C for 18 h and then calcined in a box furnace at 850 °C for 18 h (5 °C/min ramp) under air flow.
*Ink D (MnAl_{0.1}Oₓ):* Aluminum nitrate nonahydrate (43.891 g, 0.117 mol) and manganese(II) nitrate tetrahydrate (293.68 g, 1.17 mol) were dissolved in 500 mL water in a beaker under nitrogen sparge. In a separate beaker, ammonium carbonate (142.22 g, 1.48 mol) was dissolved in 660 mL water. While stirring, the metal salt solution was added slowly to the ammonium carbonate solution. The mixture was allowed to stir for 2 h. The resulting precipitate was collected by vacuum filtration and washed with deionized water. The solid was transferred to a crucible and dried in an oven at 110 °C for 18 h and then calcined in a box furnace at 950 °C for 18 h (5 °C/min ramp) under air flow and then again after grinding for another 18 h.
*Ink E* (MnOₓ):Manganese(II) nitrate tetrahydrate (293.68 g, 1.17 mol) was dissolved in 500 mL water in a beaker under nitrogen sparge. In a separate beaker, ammonium carbonate (142.22 g, 1.48 mol) was dissolved in 660 mL water. While stirring, the manganese salt solution was added slowly to the ammonium carbonate solution. The mixture was allowed to stir for 2 h. The resulting precipitate was collected by vacuum filtration and washed with deionized water. The solid was transferred to a crucible and dried in an oven at 110 °C for 18 h and then calcined in a box furnace at 450 °C for 20 h (5 °C/min ramp) under flowing air.

*Mn₃O₄ Ink:* Mn₃O₄ was purchased commercially and used as received.

*General conditions:* Metal oxide inks were formed by combining metal oxide with ink vehicle (Fuel Cell Materials) in a 1.7:1.0 mass ratio and then mixing by ball milling in a Retsch Mixer Mill MM 400 in a stabilized zirconia-lined vial of capacity of 10 mL charged with two 10 mm diameter stabilized zirconia balls for 1 h at a milling frequency of 30 Hz. Approximately 1 cm × 1 cm alumina coupons were painted with 32 mg of ink per coupon per layer and allowed to dry for 18 h under ambient conditions prior to calcination in a box furnace under static air for each successive layer. The calcining times and temperatures are given in Table 2.

Example 10A (Inventive): In this example, the alumina coupon was successively coated with Inks A, B, C, and D The calcining times and temperatures are given in Table 4. A final dark brown layer demonstrated good physical adhesion.

Example 10B (Inventive): In this example, the alumina coupon was successively coated with Inks A, B, and D as in Example 10A, but without Ink C. The calcining times and temperatures are given in Table 4. Ink layer 3 showed cracks along which pieces could be readily mechanically chipped off.

Example 10C (Comparative): In this example, the alumina coupon was coated with an ink made with Mn₃O₄. The ink was prepared and applied as in Example 10A. The calcining temperature was 1400°C for 4 h. The manganese oxide layer could readily be sloughed off of the coupon substrate as a mostly intact single sheet, leaving brown staining behind on the coupon.

Example 10D (Comparative): In this example, the alumina coupon was successively coated with an ink made with MnO. The ink was prepared and applied as in Example 10A. The calcining temperature was 1400°C for 4 h. The manganese oxide layer could readily be sloughed off of the coupon substrate as a mostly intact single sheet, leaving brown staining behind on the coupon.

**TABLE 4**

| **Example** | 10A | 10B | 10C | 10D |
|---|---|---|---|---|
| **Substrate** | Al₂O₃ | Al₂O₃ | Al₂O₃ | Al₂O₃ |
| **Layer 1** | | | | |
| Mn/Al | Ink A | Ink A | Mn₃O₄ Ink | Ink E |
| Oxide | MnAlOₓ | MnAlOₓ | | MnOₓ |
| Calcination Conditions | 1400 °C, 4 h | 1400 °C, 4 h | 1400 °C, 4 h | 1400 °C, 4 h |
| **Layer 2** | | | | |
| Mn/Al | Ink B | Ink B | None | None |
| Oxide | Mn₂AlOₓ | Mn₂AlOₓ | | |
| Calcination Conditions | 1400 °C, 4 h | 1400 °C, 4 h | | |
| **Layer 3** | | | | |
| Mn/Al | Ink C | Ink D | None | None |
| Oxide | MnAl_{0.2}Oₓ | MnAl_{0.1}Oₓ | | |
| Calcination Conditions | 850 or 1000 °C, 4 h | 950 °C, 4 h | | |
| **Layer 4** | | | | |
| Mn/Al | Ink D | None | None | None |
| Oxide | MnAl_{0.1}Oₓ | | | |
| Calcination Conditions | 950 °C, 4 h | | | |
| Adhesion | Good | Fair | Very poor | Very poor |

## Claims

1. A reverse flow reaction process, comprising:
a forward feed flow cycle comprising heating and reacting a forward flow of a feed through a flow-through reactor member to produce a first reaction product via an endothermic reaction;
a reverse feed flow cycle comprising heating and reacting a reverse flow of the feed through the reactor member in a reverse flow direction opposite the forward feed cycle to produce a second reaction product via an endothermic reaction; and
a regeneration cycle comprising heating and regenerating the reactor member with a flow of regenerant through the reactor member in the reverse flow direction.

2. The reverse flow reaction process of claim 1, further comprising parallelly orienting a plurality of the reactor members, and sequentially alternating ones of the reactor members between a forward reaction set in the forward feed flow cycle, a reverse reaction set in the reverse feed flow cycle, and a regeneration set in the regeneration cycle.

3. The reverse flow reaction process of claim 2, further comprising:
wherein the reactor members comprise first and second opposed apertures adjacent respective first and second ends of a reactor and flow passages through the respective reactor members between the first and second opposed apertures;
wherein the forward feed flow cycle comprises:
establishing the forward flow of the feed from a first feed conduit to the first apertures of the forward reaction set of the reactor members;
passing the forward flow of the feed through the reactor members in a forward direction to form a first reaction product;
establishing a flow of the first reaction product from the second apertures of the forward reaction set of the reactor members to a first reaction product conduit;
wherein the reverse feed flow cycle comprises:
establishing the reverse flow of feed from a second feed conduit to the second apertures of the reverse reaction set of the reactor members;
passing the reverse flow of feed through the reactor members in a reverse direction to form a second reaction product;
establishing a flow of the second reaction product from the first apertures of the reverse reaction set of the reactor members to a second reaction product conduit; and
wherein the regeneration cycle comprises:
establishing the reverse flow of the regenerant from a regenerant conduit to the second apertures of the regeneration set of the reactor members;
passing the regenerant through the reactor members in a reverse direction, to regenerate the reactor members;
establishing a flow of regenerant effluent from the first apertures of the regeneration set of the reactor members to an oxidant-lean conduit.

4. The reverse flow reaction process of claim 2 or claim 3, further comprising repeating the sequential alternation a plurality of times.

5. The reverse flow reaction process of any of claims 2 to 4, further comprising a purge cycle following each of the respective forward feed flow, reverse feed flow, and regeneration cycles (preferably wherein the purge cycles comprise passing an inert gas (preferably steam) through, or applying vacuum to, a respective purge set of the reactor members).

6. The reverse flow reaction process of any of the preceding claims, further comprising:
recuperating heat, from an effluent of the regenerant and from a reverse feed flow reaction product, into the forward flow of the feed; and
recuperating heat, from a forward feed flow reaction product, into the reverse flow of regenerant and the reverse flow of the feed.

7. The reverse flow reaction process of any of the preceding claims, wherein a temperature of the first reaction product exiting the reactor member(s) is no more than 200 °C (preferably no more than 150 °C, more preferably no more than 100 °C) higher than a temperature of the forward flow of feed entering the reactor member(s), and/or a temperature of the second reaction product exiting the reactor member(s) is no more than 200 °C (preferably no more than 150 °C, more preferably no more than 100 °C) higher than a temperature of the reverse flow of feed entering the reactor member(s).

8. The reverse flow reaction process of any of the preceding claims, comprising a temperature swing between cycles of no more than 50°C (preferably no less than about 25°C, more preferably the temperature swing is about 35°C).

9. The reverse flow reactor of any of the preceding claims, further comprising a balanced heat flow wherein convection in the reactor member(s) in the forward feed flow cycle matches total convection in the reactor member(s) in the reverse feed flow cycle and the regeneration cycle.

10. The reverse flow reaction process of any of claims 2 to 9, wherein a residence time of the forward flow of feed in the forward flow set of the reactor members is different (preferably shorter, more preferably about 30% shorter) than a residence time of the reverse flow of feed in the reverse flow set of the reactor members.

11. The reverse flow reaction process of any of the preceding claims, further comprising:
wherein the reactor member(s) comprise(s) active material;
wherein the feed reaction comprises reduction of the active material, reducing an active phase of the active material from an oxidized state to a reduced state;
wherein the regeneration cycle heating comprises oxidation of the active material, oxidizing the active phase from the reduced state to the oxidized state; and
wherein the regenerant comprises an oxygen-containing gas, preferably air.

12. The reverse flow reaction process of claim 11, wherein the active material comprises a transition metal oxide, preferably manganese oxide, preferably wherein the oxidized state of the transition metal oxide comprises a spinel structure.

## Patentansprüche

1. Reverse-flow-Reaktionsverfahren, umfassend:
einen Vorwärtsdurchflusszyklus, bei dem ein Vorwärtsdurchfluss eines Einsatzmaterials durch ein Durchflussreaktorelement erwärmt und umgesetzt wird, um ein erstes Reaktionsprodukt durch eine endotherme Reaktion zu erzeugen;
einen Rückwärtsdurchflusszyklus, bei dem ein Rückwärtsdurchfluss eines Einsatzmaterials durch das Reaktorelement in einem Rückwärtsfluss in umgekehrter Strömungsrichtung zu dem Vorwärtsdurchfluss erwärmt und umgesetzt wird, um ein zweites Reaktionsprodukt durch eine endotherme Reaktion zu erzeugen; und
einen Regenerationszyklus, bei dem das Reaktorelement mit einem Regenerationsmittelstrom in Rückwärtsdurchflussrichtung erwärmt wird.

2. Reverse-flow-Reaktionsverfahren nach Anspruch 1, bei dem weiterhin eine Vielzahl von Reaktorelementen parallel ausgerichtet werden und erste der Reaktorelemente sequentiell abwechselnd zwischen einem Vorwärtsreaktionssatz im Vorwärtsdurchflusszyklus, einem Rückwärtsreaktionssatz im Rückwärtsdurchflusszyklus und einem Regenerationssatz im Regenerationszyklus betrieben werden.

3. Reverse-flow-Reaktionsverfahren nach Anspruch 2, weiterhin umfassend:
wobei die Reaktorelemente erste und zweite gegenüberliegende Öffnungen neben den jeweiligen ersten und zweiten Enden eines Reaktors und Abflusskanäle durch die jeweiligen Reaktorelemente zwischen den ersten und zweiten gegenüberliegenden Öffnungen umfassen;
wobei der Vorwärtsdurchflusszyklus umfasst:
Einrichten des Vorwärtsdurchflusses des Einsatzmaterials aus einer ersten Einspeisungsleitung zu den ersten Öffnungen des Vorwärtsreaktionssatzes der Reaktorelemente;
Durchleiten des Vorwärtsdurchflusses des Einsatzmaterials durch die Reaktorelemente in Vorwärtsrichtung zur Bildung eines ersten Reaktionsprodukts;
Einrichten eines Flusses des ersten Reaktionsprodukts von den zweiten Öffnungen des Vorwärtsreaktionssatzes der Reaktorelemente zu einer ersten Reaktionsproduktleitung;
wobei der Rückwärtsdurchflusszyklus umfasst:
Einrichten des Rückwärtsdurchflusses des Einsatzmaterials aus einer zweiten Zufuhrleitung zu den zweiten Öffnungen des Rückwärtsreaktionssatzes der Reaktorelemente;
Durchleiten des Rückwärtsdurchflusses des Einsatzmaterials durch die Reaktorelemente in Rückwärtsrichtung zur Bildung eines zweiten Reaktionsprodukts;
Einrichten eines Flusses des zweiten Reaktionsprodukts von den ersten Öffnungen des Rückwärtsreaktionssatzes der Reaktorelemente zu einer zweiten Reaktionsproduktleitung; und
wobei der Regenerationszyklus umfasst:
Einrichten des Rückwärtsdurchflusses des Regeneriermittels aus einer Regenerationsmittelleitung zu den zweiten Öffnungen des Regenerationssatzes der Reaktorelemente;
Durchleiten des Regenerationsmittels durch die Reaktorelemente in Rückwärtsrichtung, um die Reaktorelemente zu regenerieren;
Einrichten eines Flusses von Regenerationsmittel aus den ersten Öffnungen des Regenerationssatzes der Reaktorelemente zu einer Leitung, die oxidationsmittelarm ist.

4. Reverse-flow-Reaktionsverfahren nach Anspruch 2 oder Anspruch 3, bei dem weiterhin der sequentielle Wechsel mehrmals wiederholt wird.

5. Reverse-flow-Reaktionsverfahren nach einem der Ansprüche 2 bis 4, das weiterhin einen Spülzyklus im Anschluss an jeden der Vorwärtsdurchfluss-, Rückwärtsdurchfluss- und Regenerationszyklen umfasst (wobei die Spülzyklen vorzugsweise das Durchleiten eines Inertgases (vorzugsweise Dampf) oder das Anlegen eines Vakuums an einen entsprechenden Spülsatz der Reaktorelemente umfassen).

6. Reverse-flow-Reaktionsverfahren nach einem der vorangehenden Ansprüche, weiterhin umfassend:
Rückgewinnen von Wärme aus einem Abfluss des Regenerationsmittels und aus einem Reaktionsprodukt des Rückwärtsflusses in den Vorwärtsfluss des Einsatzmaterials; und
Rückgewinnen von Wärme aus einem Vorwärtsfluss des Reaktionsprodukts in den Rückwärtsfluss des Regenerationsmittels und den Rückwärtsfluss des Einsatzmaterials.

7. Reverse-flow-Reaktionsverfahren nach einem der vorhergehenden Ansprüche, bei dem die Temperatur des ersten Reaktionsprodukts, das aus dem/den Reaktorelement(en) austritt, nicht mehr als 200°C (vorzugsweise nicht mehr als 150°C; besonders bevorzugt nicht mehr als 100°C) höher ist als die Temperatur des Vorwärtsflusses des Einsatzmaterials, das in das/die Reaktorelement(e) eintritt; und/oder die Temperatur des zweiten Reaktionsprodukts, das aus dem/den Reaktorelement(en) austritt, nicht mehr als 200°C (vorzugsweise nicht mehr als 150°C; besonders bevorzugt nicht mehr als 100°C) höher ist als die Temperatur des Rückwärtsflusses des in das/die Reaktorelement(e) eintretenden Einsatzmaterials.

8. Reverse-flow-Reaktionsverfahren nach einem der vorhergehenden Ansprüche, das einen Temperaturwechsel zwischen den Zyklen von nicht mehr als 50°C umfasst (vorzugsweise nicht weniger als etwa 25°C, besonders bevorzugt beträgt der Temperaturwechsel etwa 35°C).

9. Reverse-flow-Reaktionsverfahren nach einem der vorhergehenden Ansprüche, weiterhin umfassend einen ausgeglichenen Wärmeabfluss, bei dem die Wärmekonvektion in dem/den Reaktorelement(en) im Vorwärtsdurchflusszyklus der Gesamtwärmekonvektion in dem/den Reaktorelement(en) im Rückwärtsdurchflusszyklus und im Regenerationszyklus entspricht.

10. Reverse-flow-Reaktionsverfahren nach einem der Ansprüche 2 bis 9, bei dem die Verweilzeit des Vorwärtsdurchflusses des Einsatzmaterials im Vorwärtsdurchflusssatz der Reaktorelemente anders ist (vorzugsweise kürzer, besonders bevorzugt etwa 30 % kürzer) als die Verweilzeit des Rückwärtsdurchflusses des Einsatzmaterials im Rückwärtsdurchflusssatz der Reaktorelemente.

11. Reverse-flow-Reaktionsverfahren nach einem der vorangehenden Ansprüche, weiterhin umfassend:
wobei das (die) Reaktorelement(e) aktives Material umfasst (umfassen);
wobei die Einsatzmaterialreaktion die Reduktion des aktiven Materials umfasst, bei der eine aktive Phase des aktiven Materials von einem oxidierten Zustand zu einem reduzierten Zustand reduziert wird;
wobei das Erwärmen des Regenerationszyklus die Oxidation des aktiven Materials umfasst, wobei die aktive Phase aus dem reduzierten Zustand in den oxidierten Zustand oxidiert wird; und
wobei das Regenerationsmittel ein sauerstoffhaltiges Gas, vorzugsweise Luft, enthält.

12. Reverse-flow-Reaktionsverfahren nach Anspruch 11, wobei das aktive Material ein Übergangsmetalloxid, vorzugsweise Manganoxid, umfasst, wobei der oxidierte Zustand des Übergangsmetalloxids vorzugsweise eine Spinellstruktur umfasst.

## Revendications

1. Procédé de réaction à flux inversé, comprenant :
un cycle de flux d'alimentation avant comprenant le chauffage et la réaction d'un flux avant d'une alimentation à travers un élément de réacteur traversant pour produire un premier produit de réaction par une réaction endothermique ;
un cycle de flux d'alimentation inverse comprenant le chauffage et la réaction d'un flux inverse de l'alimentation à travers l'élément de réacteur dans une direction de flux inverse opposée au cycle d'alimentation en réaction avant pour produire un second produit de réaction par une réaction endothermique ; et
un cycle de régénération comprenant le chauffage et la régénération de l'élément de réacteur avec un flux de régénérant à travers l'élément de réacteur dans la direction de flux inverse.

2. Procédé de réaction à flux inversé selon la revendication 1, comprenant en outre l'orientation parallèle d'une pluralité d'éléments de réacteur et l'alternance séquentielle de certains éléments de réacteur entre un ensemble de réaction avant dans le cycle de flux d'alimentation avant, un ensemble de réaction inverse dans le cycle de flux d'alimentation inverse et un ensemble de régénération dans le cycle de régénération.

3. Procédé de réaction à flux inversé selon la revendication 2, comprenant en outre:
les éléments de réacteur comprenant des première et seconde ouvertures opposées adjacentes aux première et seconde extrémités d'un réacteur et des passages de flux à travers les éléments de réacteur entre les première et seconde ouvertures opposées ;
le cycle de flux d'alimentation avant comportant :
l'établissement du flux avant de l'alimentation à partir d'un premier conduit d'alimentation vers les premières ouvertures de l'ensemble de réaction avant des éléments de réacteur ;
le passage du flux avant de l'alimentation à travers les éléments de réacteur dans une direction avant pour former un premier produit de réaction ;
l'établissement d'un flux du premier produit de réaction à partir des secondes ouvertures de l'ensemble de réaction avant des éléments de réacteur vers un conduit de premier produit de réaction ;
le cycle de flux d'alimentation inverse comportant :
l'établissement du flux inverse d'alimentation à partir d'un second conduit d'alimentation vers les secondes ouvertures de l'ensemble de réaction inverse des éléments de réacteur ;
le passage du flux inverse d'alimentation à travers les éléments de réacteur dans une direction inverse pour former un second produit de réaction ;
l'établissement d'un flux du second produit de réaction à partir des premières ouvertures de l'ensemble de réaction inverse des éléments de réacteur vers un conduit de second produit de réaction ; et
le cycle de régénération comportant :
l'établissement du flux inverse du régénérant à partir d'un conduit de régénérant vers les secondes ouvertures de l'ensemble de régénération des éléments de réacteur ;
le passage du régénérant à travers les éléments de réacteur dans une direction inverse afin de régénérer les éléments de réacteur ;
l'établissement d'un flux d'effluent de régénérant à partir des premières ouvertures de l'ensemble de régénération des éléments de réacteur vers un conduit pauvre en oxydant.

4. Procédé de réaction à flux inversé selon l'une des revendications 2 ou 3, comprenant en outre la répétition de l'alternance séquentielle une pluralité de fois.

5. Procédé de réaction à flux inversé selon l'une quelconque des revendications 2 à 4, comprenant en outre un cycle de purge suivant chacun des cycles respectif de flux d'alimentation avant, de flux d'alimentation inverse et de régénération (de préférence, les cycles de purge comprenant le passage d'un gaz inerte (de préférence de la vapeur) à travers, ou l'application d'un vide à, un ensemble de purge respectif des éléments de réacteur).

6. Procédé de réaction à flux inversé selon l'une quelconque des revendications précédentes, comprenant en outre :
la récupération de chaleur, à partir d'un effluent du régénérant et d'un produit de réaction de flux d'alimentation inverse, vers le flux avant de l'alimentation ; et
la récupération de chaleur, à partir d'un produit de réaction de flux d'alimentation avant, vers le flux inverse de régénérant et le flux inverse de l'alimentation.

7. Procédé de réaction à flux inversé selon l'une quelconque des revendications précédentes, une température du premier produit de réaction sortant de l'élément ou des éléments de réacteur étant supérieure d'au plus 200 °C (de préférence au plus 150 °C, plus préférablement au plus 100 °C) à une température du flux avant d'alimentation entrant dans l'élément ou les éléments de réacteur, et/ou une température du second produit de réaction sortant de l'élément ou des éléments de réacteur étant supérieure d'au plus 200 °C (de préférence au plus 150 °C, encore plus préférablement au plus 100 °C) à une température du flux inverse d'alimentation entrant dans l'élément ou les éléments de réacteur.

8. Procédé de réaction à flux inversé selon l'une quelconque des revendications précédentes, comprenant une variation de température entre cycles ne dépassant pas 50 °C (de préférence pas inférieure à environ 25 °C, plus préférablement la variation de température étant d'environ 35 °C).

9. Réacteur à flux inversé selon l'une quelconque des revendications précédentes, comprenant en outre un flux de chaleur équilibré, la convection dans l'élément ou les éléments de réacteur au cours du cycle de flux d'alimentation avant correspondant à la convection totale dans l'élément ou les éléments de réacteur au cours du cycle de flux d'alimentation inverse et du cycle de régénération.

10. Procédé de réaction à flux inversé selon l'une quelconque des revendications 2 à 9, un temps de séjour du flux avant d'alimentation dans l'ensemble de flux avant des éléments de réacteur étant différent (de préférence plus court, plus préférablement environ 30 % plus court) d'un temps de séjour du flux inverse d'alimentation dans l'ensemble de flux inverse des éléments de réacteur.

11. Procédé de réaction à flux inversé selon l'une quelconque des revendications précédentes, comprenant en outre :
l'élément ou les éléments de réacteur comprenant un matériau actif ;
la réaction d'alimentation comprenant la réduction du matériau actif, réduisant une phase active du matériau actif d'un état oxydé à un état réduit ;
le chauffage du cycle de régénération comprenant l'oxydation du matériau actif, oxydant la phase active de l'état réduit à l'état oxydé ; et
le régénérant comprenant un gaz contenant de l'oxygène, de préférence de l'air.

12. Procédé de réaction à flux inversé selon la revendication 11, le matériau actif comprenant un oxyde de métal de transition, de préférence un oxyde de manganèse, l'état oxydé de l'oxyde de métal de transition comprenant de préférence une structure spinelle.
